# EUROPEAN PATENT APPLICATION

(11) **EP 3 730 938 A1**
(43) Date of publication of application: **28.10.2020**
(21) Application number: 19170486.5
(22) Date of filing: 23.04.2019
(51) Int. Cl.: G01N 33/483, G01N 33/68

(54) **NEW METHOD TO VISUALIZE DISTRIBUTION PROFILE OF PENETRATION OF COMPOUND(S) THROUGH THE NAIL**

(71) Applicant: Nestlé Skin Health SA, 1018 Lausanne (CH)
(72) Inventor: OSMAN-PONCHET, Hanan, 06600 ANTIBES (FR); KUNZE, Gernot, 3307 BRUNNENTHAL (CH)
(74) Representative: Axe PI

(57) **Abstract**

The present invention relates to a method to visualize distribution profile of penetration of compound(s) through a nail and advantageously on filed nails, and its use in particular for predicting the antifungal efficacy of topical compound(s).

## Description

The present invention relates to a new method to visualize distribution profile of penetration of compound(s) through human nails. In a further aspect, the present invention relates to the use of the method for testing topical compounds to predict their antifungal efficacy and to compare their penetration profile through the nail.

The nails are frequently the site of fungal infections (onychomycoses), particularly dermatophytic or candidal onychomycoses, or other diseases such as psoriasis or the like. Although the preferred treatment of these pathologies involves local application of pharmaceutical compositions delivered transungually, the rigid structure of the nail makes treatment difficult.

Efficacy of onychomycosis topical therapy is then highly dependent on the capacity of antifungal agents to penetrate into the nail plate and deep into the nail bed at fungistatic and fungicidal concentrations. The nail is composed of the hard and compact protein α-keratin, characterized by low permeability (Marty JP. Amorolfine nail lacquer: a novel formulation. J Eur Acad Dermatol Venereol. 1995;4:S17-S21). The dorsal layer is a thin layer of harder and particularly impermeable keratinocytes (Kobayashi Y et al., Drug permeation through the three layers of the human nail plate. J Pharm Pharmacol. 1999;51(3):271-278). Additional challenges to the penetration of topical agents into the nail for the treatment of onychomycosis include the extent of disease and nail thickening, and the presence of dermatophytoma (Carney C et a/., A new classification system for grading the severity of onychomycosis: Onychomycosis Severity Index. Arch Dermatol. 2011;147(11):1277-1282). Therefore, mechanical methods (nail abrasion, clipping and surgical removal) and chemical techniques (softening of the nail plate prior to removal of the diseased nail) are often used to debride the nail and overcome penetration difficulties prior to topical treatment (Gupta AK et a/., Improved efficacy in onychomycosis therapy. Clin Dermatol. 2013;31(5):555-563). The penetration of compounds into and through the nail plate is influenced by various factors such as the molecular weight and hydrophilicity/lipophilicity of the diffusing molecule, nature and pH of the vehicle, keratin affinity, solute charge, and the capacity to cross air-filled cavities in the diseased nail plate and/or nail bed (Murdan S. Drug delivery to the nail following topical application. Int J Pharm. 2002;236(1-2):1-26; Murdan S. Enhancing the nail permeability of topically applied drugs. Expert Opin Drug Deliv. 2008;5(11):1267-1282; and Polak A et a/., Agar sublimation test for the in vitro determination of the antifungal activity of morpholine derivatives. Mycoses. 2004;47(5-6):184-192).

Antifungal nail lacquers are considered a form of transungual drug delivery system, providing high concentration gradient and continuous release of the active agent, essential for maximal penetration (Murdan S. Drug delivery to the nail following topical application. Int J Pharm. 2002;236(1-2):1-26; Murdan S. Enhancing the nail permeability of topically applied drugs. Expert Opin Drug Deliv. 2008;5(11):1267-1282; Akhtar N et a/., Onychomycosis: Potential of nail lacquers in transungual delivery of antifungals. Scientifica (Cairo). 2016;2016:1387936; Pittrof F et a/., Loceryl nail lacquer-realization of a new galenical approach to onychomycosis therapy. Clin Exp Dermatol. 1992; 17(Suppl1):26-28).

Amorolfine 5% nail lacquer (LOCERYL® 5%, Nestle Skin Health - Galderma, Lausanne, Switzerland) is a morpholine derivative, broad-spectrum topical antimycotic, possessing concentration-dependent fungicidal and sporicidal properties (Favre B et al., Comparison of in vitro activities of 17 antifungal drugs against a panel of 20 dermatophytes by using a microdilution assay. J Clin Microbiol. 2003;41(10):4817-4819; Seidl HP et a/., Sporicidal effect of amorolfine and other antimycotics used in the therapy of fungal nail infections. Mycoses. 2015;58(10):610-619; Polak AM. Preclinical data and mode of action of amorolfine. Clin Exp Dermatol. 1992;17(Suppl 1):8-12; Polak A. Kinetics of amorolfine in human nails. Mycoses. 1993;36(3-4):101-103). It is applied once to twice weekly on the diseased nails after thorough filing of the affected areas of the nail (Reinel D et al., Comparative efficacy and safety of amorolfine nail lacquer 5% in onychomycosis, once-weekly versus twice-weekly. Clin Exp Dermatol. 1992;17(Suppl 1):44-49; Lauharanta J. Comparative efficacy and safety of amorolfine nail lacquer 2% versus 5% once weekly. Clin Exp Dermatol. 1992;17(Suppl 1):41-43). Other topical antifungals for the treatment of onychomycosis, such as naftifine hydrochloride 1% solution and ciclopirox 8% nail lacquer, need to be applied once daily and do not require nail filing (Naftifine hydrochloride 1% solution (EXODERIL®, Sandoz GmbH, Austria), Summary of Product Characteristics, June 2014; Ciclopirox 8% nail lacquer (CICLOPOLI®, Taurus Pharma, Germany), Summary of Product Characteristics, October 2017).

Common existing method to evaluate distribution profile and to measure penetration of test molecules through the nail generally consists on:
- applying test compound on the surface of nail;
- incubate for several hours;
- remove excess of the test compound on the surface of nail;
- perform horizontal slices of the nail from the top (dorsal surface) to the bottom (ventral surface) in several thin slices (10 µm), between ten and twenty slices being needed according to nail thickness;
- dissolve each slice and extract test compound using appropriate organic solvent;
- analyze each slice extract using sensitive analytical method (for example LC-MS/MS); and
- from analysis result, set up distribution profile in the nail.

For example, we know a previous study investigating the penetration kinetics of a single application of amorolfine 5% nail lacquer through nails for a period of 24 hours (Polak A. Kinetics of amorolfine in human nails. Mycoses. 1993;36(3-4):101-103). Each nail was cut out and sectioned into horizontal slices using a microtome, into at least 5 layers to 12 layers from the surface down. The number of slices varied according to the thickness and condition of the nail, layer 1 being the uppermost, and "remainder" denotes the lowermost portion which could not be further sliced. the nail samples were then weighed and treated with KOH (0.5 ml 20% wt/vol at 37°C overnight), during which time the sliced nail completely dissolved. The KOH solution was then acidified with hydrochloric acid and the amount of amorolfine present was measured microbiologically using an agar dilution technique. It was observed that, in all cases, antifungal activity was measurable even in the lower part of the nail 24 hours after application. There was an exponential relationship between the concentration of the drug and the layer of the nail. The uppermost nail layers contained approximately 100-fold higher concentration of amorolfine than the lower layers. In the lower layers, the drug concentration still exceeded the minimum inhibitory concentration (MIC) for *Trichophyton rubrum (T. rubrum).*

However, existing method using horizontal slices involves limitations due to nail curve and nail slicing: the nail plate is a convex surface and the curvature is different from nail to other. It has an impact when performing nail slices and it has an impact on the accuracy of the concentration in each slice.

Furthermore, it is impossible with such a method to obtain a distribution profile of penetration of compound(s) through human nails from only one horizontal slice. It requires at least 10 horizontal slices, i.e. 20 slices, to try to reconstitute a penetration profile of a compound through the nail with the problems related to the use of several slices and curvature of the nail indicated above.

We also know the patent document WO2013156225 which relates to penetration and distribution in the nail using autoradiography method. Following application of the radiolabeled pharmaceutical composition on the nails previously drilled to create holes in the nail plate, the nail sample is sectioned proximate the holes into a plurality of sections using a microtome or like device. The sections are on the order of micrometers thick and may be taken onto adhesive tape. The sections are preferably freeze dried to prevent decomposition during later steps. Each of the sections is then analyzed to determine a concentration of radioactivity in the section as a function of position with respect to the holes. It is preferred that quantitative whole-body autoradiography (QWBA) is used to make this determination, although other techniques for quantitatively assessing radioactivity levels in tissue may be used as well.

However, existing method involves limitations due to the use of radiolabeled compounds and manufacturing of the formulations with radiolabeled compounds.

The present inventors aim to develop a new method to visualize distribution profile of penetration of compound(s) through the nail overcoming the above drawbacks and without using radioactive labeled substance.

Namely, the present inventors have developed a method easy to use, allowing to accurately obtain nail penetration profile and visualize distribution profile of penetration of test compound(s) in the nail compared to the existing method (further excluding bias related to requiring several horizontal slicing of a convex nail or analytical method sensitivity) and offering time and resources saving.

A first object of the solution proposed by the invention is to provide a method to visualize distribution profile of penetration of compound(s) through human nail comprising the following steps of:
a) applying a test compound on the surface of a nail;
b) incubating the applied nail for at least 1 hour, preferably 24 hours at 30°C;
c) removing excess of the test compound on the surface of the applied nail;
d) performing one single thin section perpendicular to the applied nail surface;
e) analyzing the nail section using MALDI-MS imaging method; and
f) setting up distribution profile to visualize distribution profile of penetration of the test compound in the nail.

Thus, a second object is the use of the method of the invention to visualize distribution profile of penetration of a same compound on intact nail and on filed nails.

A third and final object is the use of the method of the invention to predict the antifungal efficacy of topical compound(s) and to compare their penetration profile through the nail.

For the purpose of illustrating aspects of the present invention, there are depicted in the drawings certain embodiments of the invention. However, the invention is not limited to the precise arrangements and instrumentalities of the embodiments depicted in the drawings. The invention shall be better understood when reading the following non-restricted description, with reference to the accompanying drawings, wherein:
- Figures 1 show images of nail section of untreated nail: microscopic image of nail section (Fig. 1A), MALDI image of amorolfine (Fig. 1B) and MALDI image of unknown biomarker of the nail used to show the contour of the nail (Fig. 1C);
- Figures 2 show images of nail section of intact nail treated with LOCERYL 5% nail lacquer: microscopic image of nail section (Fig. 2A), MALDI image of amorolfine (Fig. 2B) and MALDI image of unknown biomarker of the nail used to show the contour of the nail (Fig. 2C); and
- Figures 3 show images of nail section of filed nail treated with LOCERYL 5% nail lacquer: microscopic image of nail section (Fig. 3A), MALDI image of amorolfine (Fig. 3B) and MALDI image of unknown biomarker of the nail used to show the contour of the nail (Fig. 3C).

A new method to visualize distribution profile of penetration of compound(s) through human nail has been developed. The method comprises the following steps of:
a) applying a test compound on the surface of a nail;
b) incubating the applied nail for at least 1 hour, preferably 24 hours at 30°C;
c) removing excess of the test compound on the surface of the applied nail;
d) performing one single thin section perpendicular to the applied nail surface;
e) analyzing the nail section using MALDI-MS imaging method; and
f) setting up distribution profile to visualize distribution profile of penetration of the test compound in the nail.

The nail is obtained from a healthy human cadaver toenail, from onychomycosis-infected human cadaver toenail, or from finger nail clipping from alive subject, preferably a big toe.

According to an embodiment of the invention, the test compound is applied on *"ex vivo"* nails from human cadaver.

According to another embodiment of the invention, the test compound is applied *"in vivo"* on nails from alive subject before being clipped.

The nail is then preferably washed and stored at -80°C.

According to a particular embodiment of the invention, prior to application of the test compound on the surface of the nail (step a) obtained from a human cadaver, the nail is previously thawed at room temperature, immersed in deionized water, and heated at 60°C for 15 minutes, and then washed, advantageously twice, with 70% ethanol for 1 minute and sterile water.

Concerning step a), one compound per nail is preferably applied in a quantity of at least 5 µL/cm², for example 10 µL/cm² or 25 µL/cm², preferably for example 10 µL/cm², on the surface of the nail in a marked zone of at least 0,5 cm², preferably 1 cm².

The nail is then advantageously allowed to air dry for at least 5 minutes, preferably 30 minutes, after application of the test compound on the surface of the nail (step a) and before incubation (step b).

After step a), the treated nail is preferably placed in a Petri dish, on a gauze moistened with sterile water, and placed in an incubator at a temperature between 20-30°C for at least 1 hour, preferably 24 hours.

After step b), unabsorbed compound is removed using one or more cotton swab(s) wetted with an appropriate organic solvent, like aceton (step c).

After step c), the marked zone of each applied nail is excised.

The nail is then preferably embedded in 5% CMC.

Concerning step d), one single thin section perpendicular to the applied nail surface of preferably 5-20-µm section, more preferably 10-µm, is performed.

Such a nail section is advantageously performed using a cryostat set at -20°C and thaw-mounted on microscope glass slides before storing at -80°C.

According to a particular embodiment of the invention, 3 sections of 5-20-µm, preferably 10 µm, perpendicular to the same nail treated surface are performed spaced by at least 100 µm.

Before step e), the nail section is advantageously spun at 150 rpm while applying matrix solution, which consisted of 30 mg/mL 2,5-dihydroxybenzoic acid (DHB) in 90:10 methanol :water.

A volume of 300 µL matrix solution is then sprayed onto the nail section using nitrogen as a carrier gas and a syringe pump with a flow rate of 30 µL/min.

The nail section is then analyzed using a sensitive analytical method (step e).

The sensitive analytical method used is MALDI-MS imaging method, preferably with a spatial resolution of 20 x 20 µm.

The present invention also relates to the use of the method of the invention to visualize distribution profile of penetration of a same compound on intact nail and on filed nails, for example across the surface using 4 back and forth movements (8) with an emery board.

According to a particular embodiment of the invention, the method is used to evaluate the antifungal efficacy of topical compound(s).

The following examples are intended to illustrate particular embodiments of the present invention but are by no means intended to limit the scope of the present invention.

### Example 1: Method of the invention to visualize penetration and distribution of amorolfine through a nail using MALDI imaging technic

### 1- Nail treatment

Three (3) healthy toenails (big toe) from human cadaver were used through the study.

One untreated nail (intact nail, left, male) was used as control.

One intact nail (right, female) was treated with LOCERYL® 5% nail lacquer for 24 hours.

One nail (left, female) was filed and then treated with LOCERYL® 5% nail lacquer for 24 hours.

Treatment with LOCERYL® 5% nail lacquer was within a square of 1 cm using 10 µL of the lacquer leading to treatment of 10µL/cm².

At the end of treatment period, the excess of nail lacquer was removed using cotton wetted with acetone.

### 2- Tissue preparation and matrix application for MALDI

The nails were embedded in 5% CMC (Carboxymethyl cellulose) and cut in perpendicular cross sections of 10 µm thickness at -20°C using a cryomicrotome and thaw-mounted on microscope glass slides.

The nail section was spun at 150 rpm while applying matrix solution, which consisted of 30 mg/mL 2,5-dihydroxybenzoic acid (DHB) in 90:10 methanol: water.

A volume of 300 µL matrix solution was sprayed onto the nail section using nitrogen as a carrier gas and a syringe pump with a flow rate of 30 µL/min.

Detection of Amorolfine (m/z 318.2793 ± 0.002) was done by MALDI-MSI in positive ionization mode. Detection of an unknown biomarker of the nail (m/z 369.35130 ± 0.002) was used to show the contour of the nail.

MALDI images were acquired with spatial resolution of 20 × 20 µm.

### 3- Results

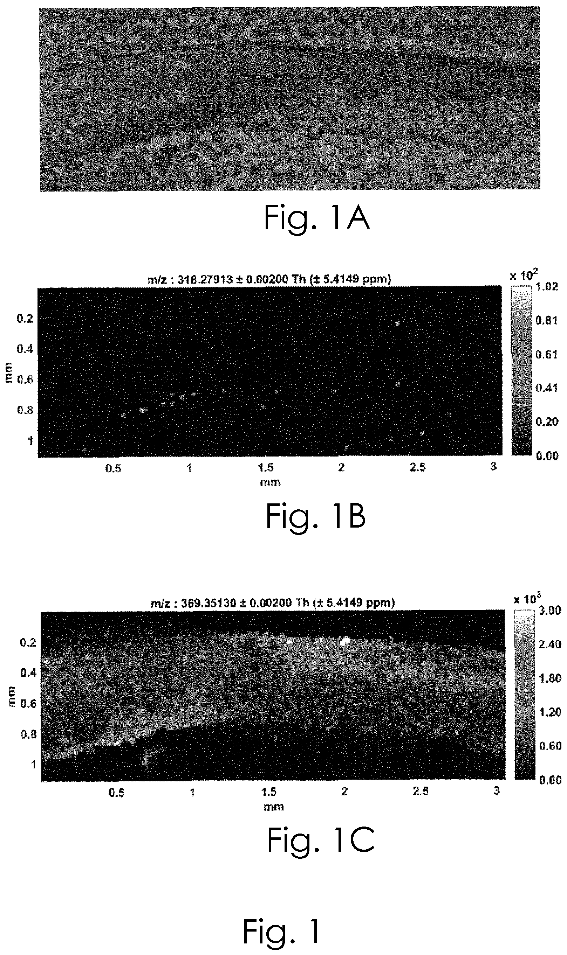

Figure 1 show MALDI images of control untreated nail.
Figure 1A (top): Microscope image of the nail section taken after MALDI imaging showing the still intact MALDI matrix (laser ablation craters are seen in an ordered array).
Figure 1B (middle): MALDI image shows no signal related to Amorolfine (m/z 318.2793 ± 0.002) which can be detected in the control untreated nail.
Figure 1C (bottom): MALDI image shows the unknown biomarker (m/z 369.35130 ± 0.002) distribution in the control untreated nail used to show the contour of the nail.

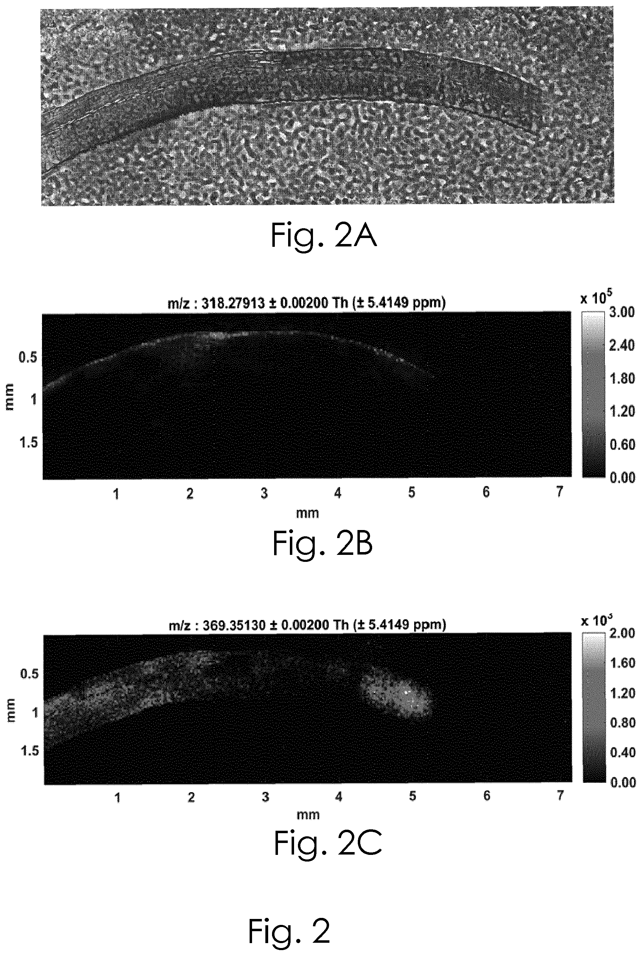

Figure 2 shows MALDI images of intact nail treated with LOCERYL® 5% nail lacquer.
Figure 2A (top): Microscope image of the nail section taken after MALDI imaging showing the still intact MALDI matrix (laser ablation craters are seen in an ordered array).
Figure 2B (middle): MALDI image shows the Amorolfine (m/z 318.2793 ± 0.002) distribution in the intact nail treated with LOCERYL® 5% nail lacquer.
Figure 2C (bottom): MALDI image shows the unknown biomarker (m/z 369.35130 ± 0.002) distribution, used to show the contour of the nail, in the intact nail treated with LOCERYL® 5% nail lacquer.

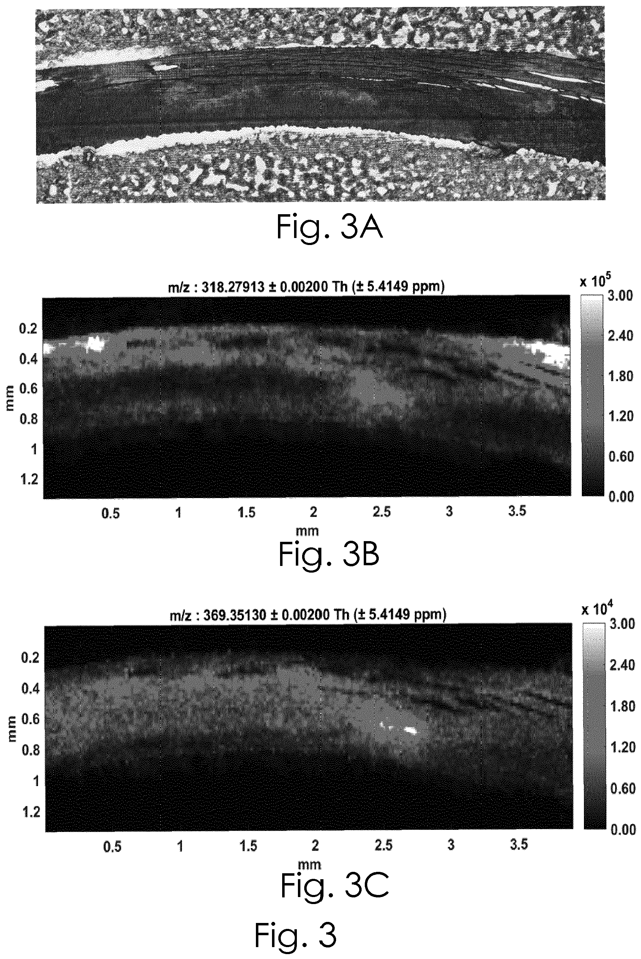

Figure 3 shows MALDI images of filed nail treated with LOCERYL® 5% nail lacquer.
Figure 3A (top): Microscope image of the nail section taken after MALDI imaging showing the still intact MALDI matrix (laser ablation craters are seen in an ordered array).
Figure 3B (middle): MALDI image shows the Amorolfine (m/z 318.2793 ± 0.002) distribution in the filed nail treated with LOCERYL® 5% nail lacquer.
Figure 3C (bottom): MALDI image shows the unknown biomarker (m/z 369.35130 ± 0.002) distribution, used to show the contour of the nail, in the filed nail treated with LOCERYL® 5% nail lacquer.

In the intact nail treated with LOCERYL® 5% nail lacquer, the signal related to Amorolfine can only be visualized on the upper layers of the nail. Conversely, in the filed nail treated with LOCERYL® 5% nail lacquer, the signal related to Amorolfine can be visualized over the entire depth of the nail. This indicates that filing the nail considerably increase the nail penetration of Amorolfine.

The unknown marker of the nail is distributed homogeneously in the nail whatever the treatment condition, indicating the specificity of the signal related to Amorolfine.

### Conclusion

The results show that filing the nail before treatment with LOCERYL® 5% nail lacquer considerably increases the penetration of Amorolfine through the nail as can be seen by MALDI imaging, and that MALDI imaging technic represents a useful tool to visualize penetration and distribution of Amorolfine through the nail.

## Claims

1. A method to visualize distribution profile of penetration of compound(s) through human nail comprising the following steps of:
a) applying a test compound on the surface of a nail;
b) incubating the applied nail for at least 1 hour, preferably 24 hours at 30°C;
c) removing excess of the test compound on the surface of the applied nail;
d) performing one single thin section perpendicular to the applied nail surface;
e) analyzing the nail section using MALDI-MS imaging method; and
f) setting up distribution profile to visualize distribution profile of penetration of the test compound in the nail.

2. The method according to claim 1, wherein the nail is obtained from a healthy human cadaver toenail, from onychomycosis-infected human cadaver toenail, or from finger nail clipping from alive subject, preferably a big toe.

3. The method according to claim 2, wherein the nail is washed and stored at - 80°C.

4. The method according to any of claims 1 to 3, wherein one compound per nail is applied in a quantity of at least 5 µL/cm² on the surface of the nail in a marked zone of at least 0,5 cm².

5. The method according to any of claims 1 to 4, wherein the nail is allowed to air dry for at least 5 minutes after application of the test compound on the surface of the nail (step a) and before incubation (step b).

6. The method according to any of claims 1 to 5, wherein after step a, the applied nail is placed in a Petri dish, on a gauze moistened with sterile water, and placed in an incubator at a temperature between 20-30°C for at least 1 hour, preferably 24 hours.

7. The method according to any of claims 1 to 6, wherein after step b, unabsorbed compound is removed using one or more cotton swab(s) wetted with appropriate organic solvent.

8. The method according to any of claims 4 to 7, wherein after step c, the marked zone of each applied nail is excised.

9. The method according to claim 8, wherein the nail is embedded in 5% CMC.

10. The method according to any of claims 1 to 9, wherein one single thin section perpendicular to the applied nail surface of 5-20-µm section, preferably 10-µm, is performed.

11. The method according to any of claims 1 to 10, wherein the nail section is spun at 150 rpm while applying matrix solution, which consisted of 30 mg/mL 2,5-dihydroxybenzoic acid (DHB) in 90:10 methanol:water.

12. The method according to claim 11, wherein a volume of 300 µL matrix solution is sprayed onto the nail section using nitrogen as a carrier gas and a syringe pump with a flow rate of 30 µL/min.

13. The method according to any of claims 1 to 12, wherein MALDI-MS imaging method is used with a spatial resolution of 20 x 20 µm.

14. Use of the method according to any of claims 1 to 13 to visualize distribution profile of penetration of a same compound on intact nail and on filed nails.

15. Use of the method according to any of claims 1 to 13 to predict the antifungal efficacy of topical compound(s) and to compare their penetration profile through the nail.
